# EUROPEAN PATENT SPECIFICATION

(11) **EP 3 518 986 B1**
(45) Date of publication and mention of the grant of the patent: **15.12.2021**
(21) Application number: 17781052.0
(22) Date of filing: 28.09.2017
(51) Int. Cl.: A61K 49/00

(54) **BIODEGRADABLE, SECOND-HARMONIC-GENERATING NANOPROBE FOR BIOMEDICAL IMAGING APPLICATIONS**
BIOLOGISCH ABBAUBARE NANOSONDE MIT ERZEUGUNG EINER ZWEITEN OBERSCHWINGUNG FÜR BIOMEDIZINISCHE BILDGEBUNGSANWENDUNGEN
NANOSONDE À GÉNÉRATION DE SECONDE HARMONIQUE BIODÉGRADABLE POUR DES APPLICATIONS D'IMAGERIE BIOMÉDICALE

(30) Priority: 29.09.2016 EP 16191538
(43) Date of publication of application: 07.08.2019
(73) Proprietor: Max-Planck-Gesellschaft zur Förderung der Wissenschaften E. V., 80539 München (DE); Pantazis, Periklis, 4104 Oberwill (CH); Sonay, Ali Yasin, New York, NY 10044 (US)
(72) Inventor: PANTAZIS, Periklis, 4104 Oberwil (CH); SONAY, Ali Yasin, New York, NY 10044 (US); CRESPY, Daniel, Wangchan Rayong 21210 (TH); LANDFESTER, Katharina, 55122 Mainz (DE)
(74) Representative: Schulz Junghans Patentanwälte PartGmbB
(86) International application number: PCT/EP2017/074709
(87) International publication number: WO 2018/060378

(56) References cited:
- WO-A1-2013/078410
- WO-A1-2016/007091
- US-A1- 2013 195 752
- P. PANTAZIS ET AL: "Second harmonic generating (SHG) nanoprobes for in vivo imaging", PROCEEDINGS OF THE NATIONAL ACADEMY OF SCIENCES, vol. 107, no. 33, 17 August 2010 (2010-08-17), pages 14535-14540, XP055049886, ISSN: 0027-8424, DOI: 10.1073/pnas.1004748107
- WILLIAM P. DEMPSEY ET AL: "SHG nanoprobes: Advancing harmonic imaging in biology", BIOESSAYS, vol. 34, no. 5, 1 May 2012 (2012-05-01), pages 351-360, XP055049813, ISSN: 0265-9247, DOI: 10.1002/bies.201100106
- Jelena Culic-Viskota ET AL: "Surface functionalization of barium titanate SHG nanoprobes for in vivo imaging in zebrafish", Nature protocols, vol. 7, no. 9 1 September 2012 (2012-09-01), pages 1618-1633, XP055345295, England DOI: 10.1038/nprot.2012.087 Retrieved from the Internet: URL:http://www.nature.com/nprot/journal/v7 /n9/pdf/nprot.2012.087.pdf
- SILVIA MARCHESAN ET AL: "The Phe-Phe Motif for Peptide Self-Assembly in Nanomedicine", MOLECULES, vol. 20, no. 11, 3 November 2015 (2015-11-03), pages 19775-19788, XP055344833, DOI: 10.3390/molecules201119658
- KUDRYAVTSEV A V ET AL: "Nonlinear Optical Properties of Triphenylalanine-based Peptide Nanostructures", RUSSIAN PHYSICS JOURNAL, CONSULTANTS BUREAU, NEW YORK, NY, BOSTON, vol. 59, no. 1, 6 May 2016 (2016-05-06), pages 8-15, XP035931196, ISSN: 1064-8887, DOI: 10.1007/S11182-016-0732-9 [retrieved on 2016-05-06]
- AMIR HANDELMAN ET AL: "Nonlinear Optical Bioinspired Peptide Nanostructures", ADVANCED OPTICAL MATERIALS, vol. 1, no. 11, 1 November 2013 (2013-11-01), pages 875-884, XP055345421, DE ISSN: 2195-1071, DOI: 10.1002/adom.201300282
- DAVIDE STAEDLER ET AL: "Cellular uptake and biocompatibility of bismuth ferrite harmonic advanced nanoparticles", NANOMEDICINE: NANOTECHNOLOGY, BIOLOGY AND MEDICINE, vol. 11, no. 4, 1 May 2015 (2015-05-01), pages 815-824, XP055344899, NL ISSN: 1549-9634, DOI: 10.1016/j.nano.2014.12.018
- YANG DU ET AL: "GX1-conjugated poly(lactic acid) nanoparticles encapsulating Endostar for improved in vivo anticolorectal cancer treatment", INTERNATIONAL JOURNAL OF NANOMEDICINE, vol. 10, no. 1, 28 May 2015 (2015-05-28), pages 3791-3802, XP055344992, DOI: 10.2147/IJN.S82029
- SERRA-GÓMEZ R ET AL: "Cyclodextrin-grafted barium titanate nanoparticles for improved dispersion and stabilization in water-based systems", JOURNAL OF NANOPARTICLE RESEARCH, KLUWER ACADEMIC PUBLISHERS, DORDRECHT, NL, vol. 18, no. 1, 20 January 2016 (2016-01-20), pages 1-16, XP035925090, ISSN: 1388-0764, DOI: 10.1007/S11051-015-3321-X [retrieved on 2016-01-20]

## Description

### Specification

The invention relates to a second-harmonic generating, water-suspensable nanoparticle, a method for preparing an aqueous solution of second-harmonic generating nanoparticles, a method for second-harmonic generation imaging and the use of such second-harmonic generating nanoparticles for imaging applications.

In optical imaging there are many techniques to generate and acquire an optical signal. One of these imaging techniques is the so-called second-harmonic generation imaging. Second-harmonic generation imaging is based on second-harmonic generation (SHG).

SHG is a nonlinear optical scattering-process, in which due to a non-linear susceptibility term of the scattering material, two photons with the same frequency result in a single, new photon with twice the energy, and therefore twice the frequency of the two initial photons.

This effect is used in imaging applications like SHG imaging. In these applications the SHG probes respond with an SHG signal under intense illumination. As the SHG probe signal has a narrow signal profile and twice the wavelength of the excitation light, the SHG signal from the SHG probes can be detected with minimal background.

Furthermore, due to the scattering nature of SHG, SHG imaging does not suffer bleaching or fluorescence intermittency of the probe, as the SHG probe is not excited to higher energy levels (like in fluorescence imaging) from where bleaching and intermittency of the probe occurs.

SHG probes are known in the state-of-the-art, which are made of inorganic material that is not biodegradable, and therefore limiting the prospect of having them in clinical use or under clinical development (US 8945 471, US 9221 519).

Pantazis et al. (2010) PNAS USA 107,14535-14540 discloses SHG-generating probes made of barium titanate (BaTiO3) particles in the range of 30 nm.

Dempsey et al. (2012) BioEssays 34,351-360 reviews the application of inorganic SHG-generating nanoparticles for in vivo imaging applications.

WO 2013/078410 A1 as well as Culic-Viskota et al. (2012) Nature prot. 7,1618-1633 disclose a coating of a barium titanate SHG-generating particle with a biocompatible mPEG and biotin layer.

Efforts have been made to prepare biocompatible SHG probes using peptides, however an inherent problem during preparation is that oligopeptides self-assemble to SHG structures only in organic solvents [1]-[3] that aggregate in aqueous solutions, rendering them useless for most biological or medical applications.

For example, WO 2016/007091 A1 discloses that certain peptides may form structures that generate a SHG signal upon illumination, particularly wherein said peptides comprise amino acids arranged in a decreasing or increasing order of sizes. Coating of these peptide structures is not possible. A detailed discussion of these peptides is provided within the following specification.

Marchesan et al. (2015) Molecules 20, 19775-19788 discloses that diphenyl peptides exhibit SHG under certain circumstances.

Similarly, Kuoryavtsev et al. (May 2016) Russ. Phys. J. 59,8-15. discloses that triphenylalanine exhibits SHG properties.

Handelman et al. (2013) Adv. Opt. Mat. 1, 875-884. discloses that SHG signals have been detected in aggregates comprising diphenylalanine, cyclic diphenylalanine, and triphenylalanine in optical microscopy. Therefore, the problem underlying the present invention is to provide a water-suspensable, biocompatible, biodegradable SHG probe and a method for preparing an aqueous suspension comprising said SHG probes.

This problem is solved by a second-harmonic generating nanoparticle having the features of claim 1, a method having the features of claim 9 and 14 as well as a use for such a nanoparticle in imaging applications having the features of claim 15. Preferred embodiments are stated in the sub claims.

According to claim 1, a biodegradable, biocompatible, water-suspensable nanoparticle, for generating a second-harmonic light signal upon illumination with light, comprises
- a shell layer comprising or consisting of a biodegradable polymer, wherein the shell layer encloses
- a plurality of oligopeptides, wherein the plurality of oligopeptides is structured such that a second-harmonic light signal is generated upon illumination of the nanoparticle with light.

Such a nanoparticle advantageously solves the problem according to the invention, as it is water-soluble and biocompatible.

The water-suspensability of the nanoparticles is particularly achieved by the shell layer that comprises a biodegradable polymer, wherein the shell layer provides water-suspensability to the water-insoluble or not-well soluble, structured plurality of oligopeptides.

Another advantage of the nanoparticle according to the invention is that the nanoparticle does not tend to aggregate with other nanoparticles of the same kind in an aqueous solution, wherein the pure oligopeptides suspended in an aqueous solution might aggregate over time.

Self-assembling oligopeptides can form large scale assemblies that can generate SHG signal, as the orientation of the assembly lacks inversion symmetry. The SHG signal intensity is proportional to the number of peptides employed for the assembly, which is related to the size of the assembly.

Consequently, smaller nanoparticles particularly generate a smaller SHG signal. This invention provides a biodegradable, biocompatible, water-suspensable nanoparticle, particularly in the diameter range of 50 nm to 200 nm, that surprisingly does generate a sufficiently high SHG signal for example for use in microscopy applications. Large-scale assemblies, as for example disclosed in [4] (particle sizes ~1 µm to 10 µm), typically need to satisfy phase-matching conditions and their signal propagates predominantly in the forward direction, which limits the imaging capabilities of large assemblies. Moreover, these large-scale assemblies exhibit a particularly broad size range is due to the dynamic nature of the assembly process, which can assemble and disassemble in response to environmental changes.

Given the preference of oligopeptides to form aggregates, the nanoparticle according to the invention solves this problem by providing a coating that ensures that the oligopeptide assemblies do not aggregate and growth is restricted. This way, a precise control of the size range and distribution of the nanoparticles is achieved. Furthermore, suitable oligopeptides for the nanoparticle that generate an SHG signal upon illumination particularly comprise or consist of peptides that self-assemble into beta sheets and π-π stackings through crystallization.

In contrast, the example peptide LIVAGK disclosed in [4] relies on decreasing amino acid sizes within the peptide sequences, which do not self-assemble or generate SHG signal after encapsulation.

Furthermore, the peptides disclosed in [4] require a specific peptide concentration and pH value in order to self-assemble. Thus, the assembly process of peptides in [4] is vulnerable to environmental changes. Upon dilution or PH-change these assemblies disassemble rapidly. The instability of the peptide assemblies taught in [4] render them impractical for any tracking experiment within cells and tissues, where imaging probes typically encounter lower pH values for an extended period of time.

In contrast, the nanoparticles according to the invention remain stable under such environmental changes.

Given that the peptide assemblies in [4] are not coated with any polymer, any attempt to functionalize and target this assembly to a cell or protein of interest would necessitate adding functional groups on the peptides, which would disrupt the assembly process.

The shell layer of the nanoparticle allows the nanoparticle i) to be targeted to sites of interest with great precision and ii) to protect the assembled peptides from dissociation or disassembly. Hence, the nanoparticles according to the invention are particularly applicable as imaging agents in various biomedical applications.

specifies a restricted sequence order in order to generate the SHG-generating peptides. Given that the SHG signal was measured in a hydrogel, such restriction is necessary. In contrast, the oligopeptides suitable for the nanoparticles according to the invention, do not suffer from such restrictions and their self-assembly particularly depends on their crystal structure as opposed to their shape.

This difference particularly shows that the SHG generating mechanism in [4] is different to the SHG generation of the nanoparticles according to the invention as will be explained in the following.

The peptides disclosed in [4] require certain shapes and sizes as well as sequences to generate an SHG signal. This indicates that their crystallisation plays little role in SHG generation.

SHG signal generation of a nanoparticle according to the invention particularly relies on SHG generated by crystal unit cells.

The shell layer might not necessarily be understood as a complete and tight coating of the oligopeptides, but it might comprise pores and openings, through which particularly an organic solvent can evaporate. The shell layer or parts of the shell layer particularly extend to the inner part of the nanoparticle, wherein other parts of the layer might extend to the environment of the nanoparticle, e.g. into an aqueous solution. The exact structure of the shell layer is of no great importance, as long as it renders the nanoparticle water-suspensable, biodegradable and non-aggregating.

It is the structured plurality of oligopeptides in the nanoparticle that gives rise to the SHG signal upon illumination with light. The capability of the structured plurality of oligopeptides of generating a second-harmonic signal in turn roots in the lack of the inversion symmetry of the structured plurality of oligopeptides. Thus, without a structured plurality of oligopeptides or with a centrosymmetric structure of oligopeptides no appreciable SHG signal can be generated.

A nanoparticle according to the invention has a diameter ranging particularly between 10 nm and 5 µm, particularly between 50 nm and 1 µm, more particularly between 50 nm and 500 nm.

Furthermore, a nanoparticle according to the invention is particularly capable of generating a third-harmonic light signal upon illumination with light, wherein the third-harmonic light comprises three-times the energy than the illumination light.

### "Biocompatibility"

A biocompatible nanoparticle in the context of the present invention is particularly non-toxic to biological tissue, cells or a living body, as long as it does not comprise additional and specific epitopes or substances that are designed for triggering for example cell death or for altering cellular signalling pathways.

### "Biodegradable"

A biodegradable nanoparticle according to the invention refers the property of the nanoparticle of being degradable particularly by specific enzymes, bacteria, fungi or cells.

Illustrative biodegradable materials suitable for use in the practice of the invention include naturally derived polymers, such as acacia, gelatin, dextrans, albumins, alginates/starch, and the like; or synthetic polymers, whether hydrophilic or hydrophobic.

As used herein, the terms "biodegradable" and "biocompatible" therefore denote any synthetic or naturally-derived material that is known as being suitable for uses in the body of a living being, i.e., is particularly biologically inert and physiologically acceptable, non-toxic, and, in the context of the present invention, is biodegradable in the environment of use, i.e., can be resorbed by the body or degraded by specific enzymes or bacteria.

### "Oligopeptide"

An oligopeptide consists of at least two amino acids and comprises particularly less than 100 amino acids that are chemically linked. Particularly oligopeptides consisting of two, three, four, five, six or seven amino acids are suitable for the invention, as long as they are capable of forming a structured plurality that generates an SHG signal when comprised by the nanoparticle and when illuminated with light.

It is noted that not all structured pluralities of oligopeptides are capable of generating a second-harmonic signal, even though theoretically they should do so. Thus, each different oligopeptide has to be tested for the SHG property separately. Furthermore, it is noted that even if the oligopeptides assemble in a second-harmonic generating structure, it is observed that after preparation of the nanoparticle according to the invention, said nanoparticle might not generate the SHG signal anymore. Also here, a thorough testing of various oligopeptides is necessary.

### "Structured"

The term "structured" refers to the fact, that the plurality of oligopeptides is arranged at least area by area in an assembly that exhibits a certain regularity or repeated pattern and that the plurality of structured oligopeptides is particularly not arranged in a random coil configuration. Nonetheless it is possible that a fraction of enclosed oligopeptides in the nanoparticle is not adopting a structured configuration and does not produce an SHG signal upon illumination. This fraction is then simply not considered to be part of the structured plurality of oligopeptides.

Furthermore, the structured oligopeptides are particularly not arranged in a lattice formed exclusively by chemical bonds, such as covalent bonds.

As stated above, the structured plurality of peptides is particularly lacking inversion symmetry, such that the structured plurality of oligopeptides is capable of second-harmonic generation.

The structured plurality of oligopeptides generates a second-harmonic signal upon illumination with light, wherein the excitation light comprises preferably wavelengths in the range of 400 nm to 2000 mm.

The SHG strength or SHG susceptibility of the nanoparticle particularly depends on the number and assembly orientation of the structured oligopeptides comprised by the nanoparticle. The structured plurality of oligopeptides particularly comprises more than 100 oligopeptides.

According to another embodiment of the invention, the plurality of oligopeptides comprises or consists of self-assembling oligopeptides, wherein the plurality of structured oligopeptides is arranged in a self-assembled structure.

This embodiment is particularly advantageous as the preparation of nanoparticles is greatly simplified by using self-assembling oligopeptides.

### "Self-assembling"

The term "self-assembling" in the context of the invention refers to the property of the oligopeptides that the oligopeptides assemble in predefined structures or assemblies, wherein structure of the self-assembly particularly depends on the specific amino acid sequence of the oligopeptides and/or the specific mixture or mixing proportion of different oligopeptides as well as the solvent properties. The self-assembling process of the oligopeptides is particularly triggered by the oligopeptide concentration.

An example of a self-assembling oligopeptide is the tripeptide Phe-Phe-Phe. Said tripeptide, triphenylalanine, is capable of self-assembling into nanorods.

According to another embodiment of the invention, the plurality of oligopeptides is crystalized in crystal unit cells, wherein the SHG signal is generated from the crystal unit cells upon illumination of the nanoparticle. According to another embodiment of the invention the plurality of oligopeptides comprises or consists of at least one of:
- cyclo(-D-Trp-Tyr), wherein "D-Trp" refers to the D-amino-acid form (D enantionmer) of Trp,
- Trp-Phe,
- Phe-Phe-Phe,
- Phe-Phe,
- Ala-Ala-Ala-Ala-Ala (SEQ ID NO 01),
- cyclo(-Phe-Phe),
- Leu-Phe, and/or
- Leu-Leu,
wherein three-letter codes are used to refer to the specific amino acid sequence of the respective oligopeptide. The prefix "cyclo" indicates that the oligopeptide exhibits a cyclic structure. These oligopeptides are capable to self-assemble in a structure that is capable of second-harmonic generation and maintaining its second-harmonic generating property also when enclosed by the shell layer and when the nanoparticle is suspended in an aqueous solution.

According to another embodiment of the invention the plurality of oligopeptides comprises at least one structured and shape-persistent region exhibiting a high degree of internal order. Said shape-persistent region is capable of second-harmonic generation.

The degree of internal order of the structured plurality has to be so high that an SHG signal is generated upon illumination of the nanoparticle. Thus, ideally all enclosed oligopeptides are part of a particularly self-assembled structure, wherein also assemblies are included in the meaning of the invention, where the plurality of structured oligopeptides is assembled in a plurality of structures, wherein at least one of these structures generates a second-harmonic signal upon illumination. Thus, particularly also nanoparticles comprising a plurality of structured pluralities of oligopeptides, e.g. a plurality of nanorods or any other second-harmonic generating structure are to be summarized under the claimed invention.

These particularly identical structures might have different orientations within the nanoparticle, which is particularly advantageous with regard to its SHG susceptibility. A high degree of internal order refers particularly to the fact that the majority of oligopeptides are part of a structured plurality of oligopeptides and particularly only less than half of the enclosed oligopeptides are in an un-ordered state, such as a random coil configuration.

A high degree of internal order also refers to the fact that particularly more than 50% of the structured plurality of oligopeptides are ordered in a non-centrosymmetrical structure. All centrosymmetrically structured pluralities, such as for example a random coil configuration, lead to a cancellation of the SIHG signal due to destructive interference.

### "shape-persistent"

The term "shape-persistent" refers to the property of the nanoparticles, that the particularly self-assembled structures do not spontaneously change their shape or organization, but generally maintain their shape until the nanoparticle is degraded. According to another embodiment of the invention the plurality of structured oligopeptides is structured by means of non-covalent interactions, particularly by hydrophobic interactions and/or hydrogen bonds. Structures formed by non-covalent interactions are particularly useful for preparing the nanoparticle according to the invention, as no further chemical reactants are needed in order to obtain such particularly self-assembling structures. Furthermore, these structures tend be biodegradable.

According to another embodiment of the invention, the biodegradable polymer is one of:
- a poly(L-lactide) (PLLA) (CAS Nr:26100-51-6),
- a polyglycolide and a polylactic polyglycolic copolymer,
- a poly(ε-caprolactone)-polyether (CAS-Nr: 24980-41-4),
- a polycaprolactone,
- a poly[(D,L-lactide)-co-glycolide] (PLGA),
- a polyacrylamide,
- a poly(orthoester),
- a biodegradable polyurethane,
- a polycyanoacrylate polymer,
- a poly(y-glutamic acid) (γ-PGA) (CAS-Nr: 25736-27-0),
- a phenylalanine ethyl ester.

These polymers are particularly suitable for forming a biodegradable and biocompatible shell layer of the nanoparticle.

According to another embodiment of the invention, the nanoparticle is digestable by an enzyme, a proteinase, particularly by proteinase K (CAS-Nr: 39450-01-6), particularly after removing any surfactant. Therefore, the nanoparticles according to the invention are particularly biodigestable. This digestion might occur at a slower rate than a digestion of oligopeptides alone, i.e. without a polymer shell.

The property of being biodigestable is particularly important and advantageous when the nanoparticle is to be used in biological imaging or medical applications.

According to another embodiment of the invention the nanoparticle particularly the polymer comprises a fluorescent compound, wherein said fluorescent compound is particularly a covalently linked fluorescent dye. A nanoparticle comprising a fluorescent compound enables the co-localization of the fluorescence signal and the SHG signal stemming from the same nanoparticle and thus allows the monitoring of the preparation process of the nanoparticle.

The problem according to the invention is also solved by a method for preparing an aqueous suspension of biodegradable, water-suspended nanoparticles, wherein the nanoparticles generate a second-harmonic signal upon illumination with light, comprising the steps:
- providing an organic phase comprising an organic, particularly water-immiscible, solvent with oligopeptides and biodegradable polymers and/or monomers, wherein the monomers form the polymer when polymerized, wherein the oligopeptides in each nanoparticle are assembled in at least one structure once the nanoparticle is prepared, wherein said structure generates a second-harmonic signal when illuminated with light,
- providing a continuous aqueous phase comprising an aqueous solution with a particularly hydrophilic surfactant,
- preparing a miniemulsion of the organic phase and the aqueous phase by mixing the organic phase and the aqueous phase, wherein the miniemulsion comprises micelles or droplets, particularly with a diameter between 10 nm and 5 µm, of the organic phase emulsified in the aqueous phase,
- removing the organic solvent from the miniemulsion such that the second-harmonic generating nanoparticles are formed.

According to the definition of the UIPAC, a miniemulsion is an emulsion in which the particles or micelles of the dispersed phase have diameters in the range from approximately 50 nm to 1 µm. In the case of the present invention, the organic phase is the dispersed phase, the continuous phase corresponds to the aqueous phase and also an emulsion with micelle sizes up to 5 µm is referred to as a miniemulsion.

The oligopeptides assemble in the structured plurality of oligopeptides during preparation of the nanoparticle. The assembling is particularly starting when the micelles have formed in the miniemulsion, as the local concentration of the oligopeptides in the micelles is then high enough to trigger the assembling process. The assembling is particularly also happening during evaporation of the organic solvent from the miniemulsion, as the concentration during evaporation is increasing even more.

Given the preference of oligopeptides to form aggregates, the method according to the invention ensures that the oligopeptides do not aggregate and growth is restricted, once the oligopeptides are coated with the shell layer. This way, a precise control of the size and the size distribution of the nanoparticles according to the invention is achieved.

According to another embodiment of the invention the organic phase contains mixed stabilizers, for example an ionic surfactant, such as sodium dodecyl sulfate (n-dodecyl sulfate sodium) and a short aliphatic chain alcohol (referred to as co-surfactant) for colloidal stability, or a water-insoluble compound, such as a hydrocarbon (referred to as a co-stabilizer) limiting diffusion degradation. By adding a mixed surfactant, a co-surfactant and/or co-stabilizer the mini-emulsion according to the invention can be stabilized for several days.

The miniemulsion can be prepared by applying shear-forces to the mixed organic and aqueous phase.

According to another embodiment of the invention the miniemulsion is prepared by applying shear-forces to the organic phase and to the aqueous phase, particularly after mixing the two phases coarsely, particularly by shaking, wherein the shear-forces are applied by sonication.

According to another embodiment of the invention, the organic solvent is removed by evaporating the organic solvent from the miniemulsion, wherein evaporation is particularly achieved by heating and/or by reducing the surrounding atmospheric pressure.

The organic solvent can be removed to great extent from the miniemulsion by evaporation, such the miniemulsion transforms in an aqueous suspension comprising the aqueous, continuous phase and the water-suspended, biodegradable nanoparticles. Thus, the miniemulsion is not an emulsion anymore after the organic solvent is removed, but it is converted in an aqueous suspension of nanoparticles.

According to another embodiment of the invention, the organic solvent is chloroform, the oligopeptide is or comprises the tripeptide Phe-Phe-Phe and the polymers comprise or consist of Poly-Lactic Acid or the monomers comprise or consist of lactat and the aqueous solution comprises sodium dodecyl sulfate (n-dodecyl sulfate sodium) as surfactant.

Nanoparticles made from this composition are robust and reliable in preparation.

According to another embodiment of the invention the monomers are polymerized in a polymerization step.

This step might be carried out simultaneously or subsequently to the removal of the organic solvent. The polymerization step is providing increased stability for nanoparticles that otherwise would consist only of monomers.

The problem according to the invention is further solved by a method for second-harmonic generation imaging of a sample obtained from a person or an animal comprising a nanoparticle according to the invention, comprising the steps of:
- providing a sample comprising at least one nanoparticle according to the invention, wherein the sample is particularly a biological sample such as cells, a tissue preparation, tissue or a living organism,
- illuminating the sample with light comprising a first wavelength, particularly 1064 nm,
- detecting light at half the wavelength of the first wavelength, and particularly filtering the illumination light.

The at least one nanoparticle is particularly provided to the sample after the sample is obtained from the person or animal.

The problem according to the invention is further solved by a use of the nanoparticle according to the invention, in second-harmonic generation imaging of biological samples, cells, tissue preparations, tissue or living organisms, by applying the method for second-harmonic imaging.

Also here, the biological samples, cells tissue preparations, tissue or living organisms are particularly obtained from a person or an animal prior to the provision of nanoparticle to the sample and particularly prior to imaging.

Further features and advantages of the invention shall be described by means of a detailed description of embodiments with reference to the Figures, wherein it is shown in
Fig. 1 a schematic drawing of the preparation of a nanoparticle and a nanoparticle according to the invention;
Fig. 2 second-harmonic generation images of nanoparticles according to the invention;
Fig. 3 a schematic view of the self-assembling process of triphenylalanine;
Fig.4 a time series of second-harmonic generation images of self assembling triphenylalanine;
Fig. 5 a schematic drawing of a digestion of a self-assembled plurality oftriphenylalanine by proteinase K;
Fig. 6 a time series of second-harmonic generation images of a digestion ofself-assembled triphenylalanine;
Fig. 7 a time series of second-harmonic generation images of nonaggregating nanoparticles according to the invention; and
Fig. 8 molecular structures of cyclic oligopeptides that are suitable for assembling in a second-harmonic generating structure in thenanoparticle;
Fig. 9 SHG images for comparison of the methods and particles known from the state of the art with the nanoparticles according to the invention;
Fig. 10 SHG images showing the disassembly of oligopeptide assemblies known from the state of the art with regard to dilution;
   - Fig. 11: SHG images showing the disassembly of oligopeptide assemblies known from the state of the art with regard to the pH-value of the solvent;
   - Fig. 12: SHG images showing the stability of nanoparticles according to the invention with regard to dilution;
   - Fig. 13: SHG images showing the stability of nanoparticles according to the invention with regard to the pH-value of the solvent.

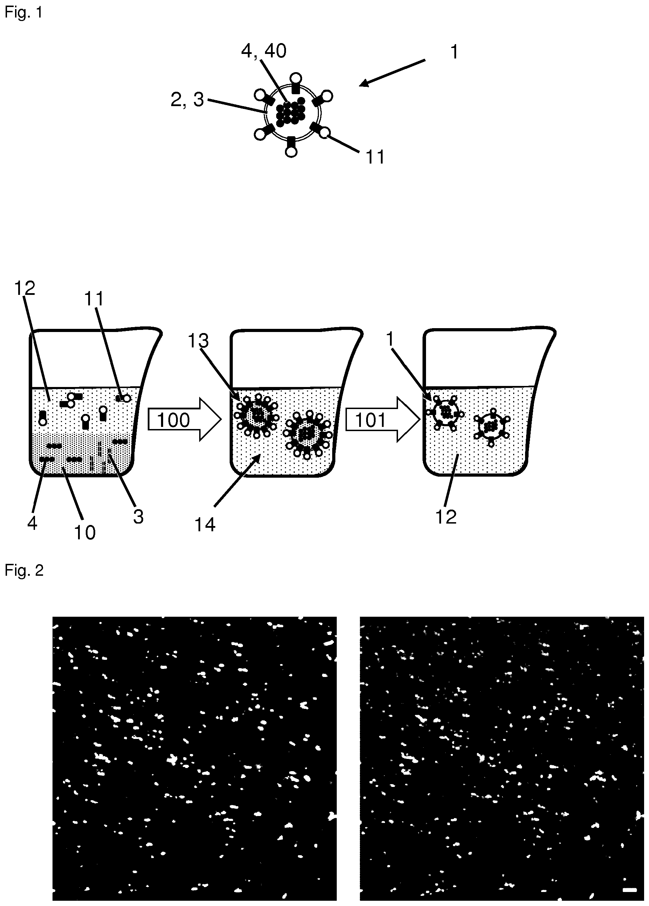

Fig. 1 schematically shows the preparation steps for obtaining a nanoparticle 1 according to the invention.

In step A) a continuous phase, here the aqueous phase 12 comprising water and a surfactant 11 is provided. The continuous phase 12 rests on top of a dispersed phase, here the organic phase 10, which comprises chloroform, a preformed polymer 3, in this case PLLA, and oligopeptides 4, in this case triphenylalanines 41, capable of self-assembling into a second-harmonic generating structure, particularly to a nanotube or a nanorod, that generates a second-harmonic signal upon illumination with light. In this example the polymer 3 is linked to a fluorescent dye, in the present case to Alexa 488, such that co-localization of the fluorescence signal and the second-harmonic signal becomes possible. This is particularly advantageous to monitor a successful preparation of second-harmonic generating nanoparticles 1 - a co-localized signal indicates that there are nanoparticles 1 suspended in the solution comprising polymer 3 and oligopeptides 4 assembled in a second-harmonic generating conformation.

In step B) the continuous phase 12 and the dispersed phase 10 are mixed together and sonicated 100 for several minutes such that micelles 13 comprising a plurality 40 of structured oligopeptides 4, the polymer 3 and the organic solvent are formed. Such emulsion is referred to as a miniemulsion 14.

The oligopeptides 4 are located on the inside of the micelles 13, where the organic solvent is encapsulated by the polymer 3. The polymer 3 thus forms a shell 2 for the oligopeptides 4. The micelles 13 are dispersed in the continuous phase 12.

The miniemulsion 14 obtained by the sonication 100 step B) is then heated (step C), such that the organic solvent evaporates 101 from the micelles 13. Once evaporated, the micelles 13 are now solid nanoparticles 1 suspended in the continuous phase 12. The nanoparticles 1 comprise the polymer shell 2 with a fluorescent dye, wherein the polymer shell 2 encapsulates the self-assembled oligopeptides 4. Upon illumination the nanoparticle 1 will generate a second-harmonic signal from the illumination light due to the self-assembled structured plurality 40 of oligopeptides 4.

One important property of these nanoparticles 1 is that besides being capable of generating a second-harmonic signal, said nanoparticles 1 also have the property that they do not aggregate over time. This is particularly useful for targeting and imaging applications that require inert probes for aqueous solutions.

As the polymer shell 2 is biodegradable the nanoparticles 1 themselves are biodegradable.

Fig. 2 shows images of second-harmonic generating nanoparticles 1 that comprise a fluorescently labelled polymer 3 according to the invention. On the left panel the SHG image is depicted, wherein on the right panel of Fig. 2 the fluorescence image is shown. The high degree of co-localization of the SHG signal and the fluorescence signal can be readily seen. The high degree of co-localization in turn indicates a successful formation of second-harmonic generating nanoparticles 1 comprising a polymer shell 2.

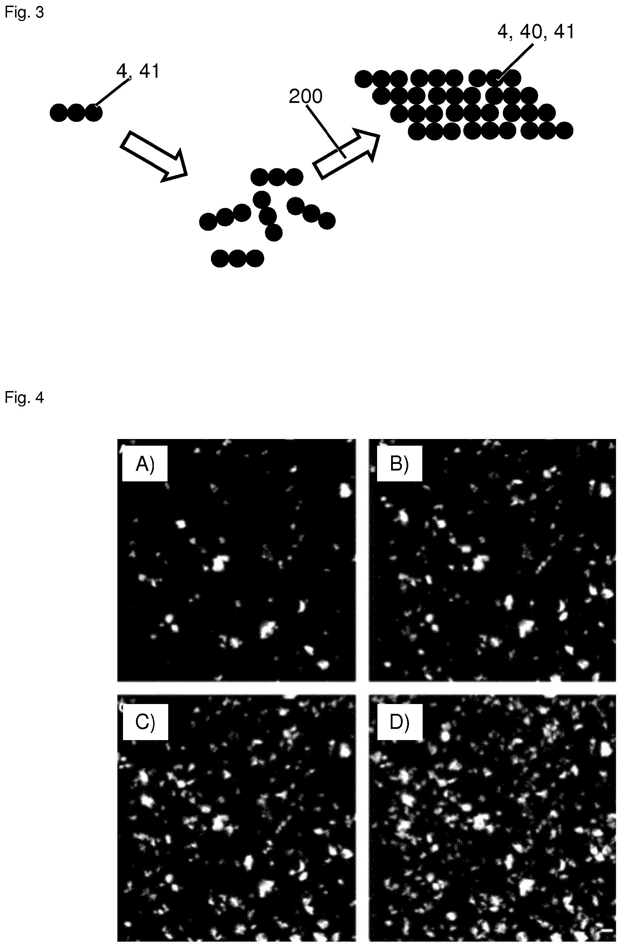

Fig. 3 schematically shows how a plurality 40 of oligopeptides 4, in this case triphenylalanines 41 (Phe-Phe-Phe), self-assemble 200 to a structure, namely a nanorod, that due to its lack of inversion symmetry is capable of second-harmonic generation.

Fig. 4 shows a time series of SHG images of triphenylalanine 41 oligopeptides 4 dissolved deionized water containing 1% Pluronic surfactants. The time series shows the effect of aggregation over time of uncoated nanoparticles, i.e. nanoparticles that do not comprise a polymer shell. Image A) has been taken at time zero minutes, image B) has been taken after 10 minutes, image C) has been taken after 20 minutes and image D) has been taken 40 minutes after dissolving the oligopeptides.

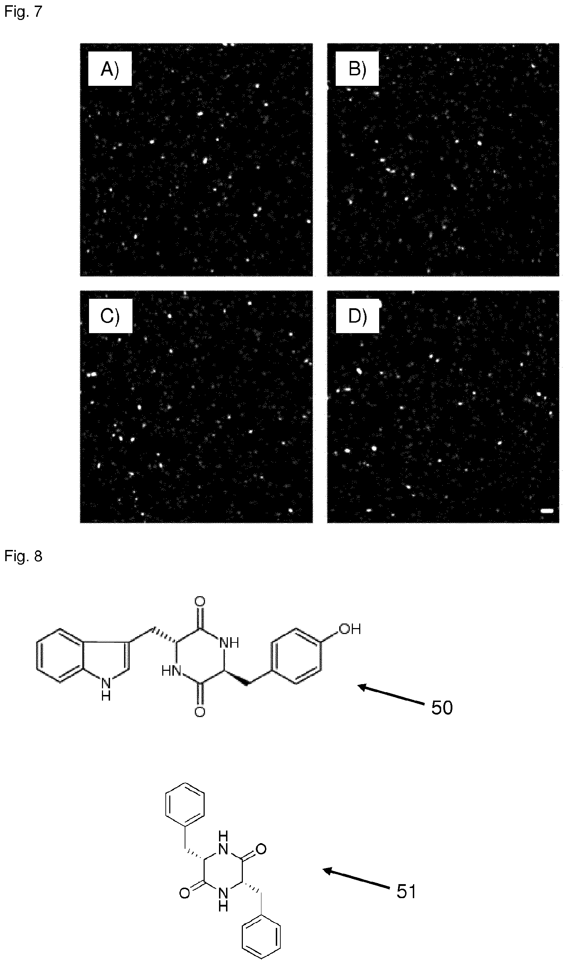

As can be seen, the overall SHG signal increases, which indicates the formation of more and larger self-assembled nanostructures that are capable of second-harmonic generation. It also indicates that the cluster size is increasing with time, which points to a progressing aggregation of self-assembled structures. Therefore the polymer shell is particularly advantageous in order to inhibit a progressing aggregation that would take place otherwise. The aggregation-inhibiting effect of the polymer shell 2 is shown in Fig. 7.

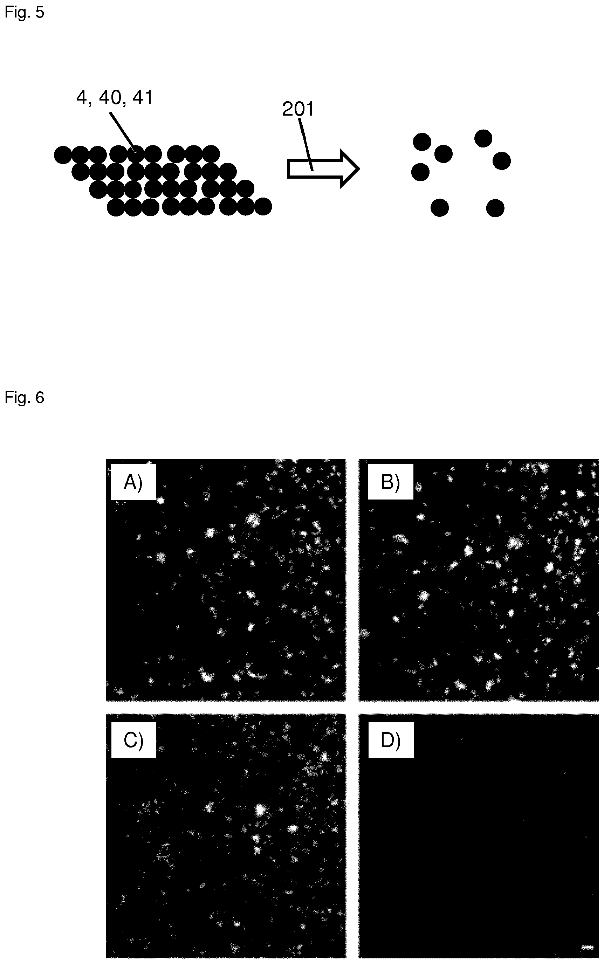

Fig. 5 shows a schematic drawing of a digestion 201 of a self-assembled structure of oligopeptides 4 comprising triphenylalanine 41, wherein the structures are not enclosed by a polymer shell 2. The digestion 201 is facilitated by a proteinase K.

The proteinase K digests the self-assembled structure into single phenylalanines.

In Fig. 6 the corresponding SHG images of a digestion 201 of self-assembled structures of triphenylalanines 41 suspended in aqueous solution with proteinase K is shown. Here, image A) has been taken at time 0 minutes, image B) at 20 minutes, C) at 40 minutes and image D) after 60 minutes after adding proteinase K to the solution. As can be readily seen, the SHG signal generated by the self-assembled structures of triphenylalanines 41 is decreasing over the course of time, indicating the digestion 201 or decomposition of the structure.

Fig. 7 shows that the nanoparticles 1 comprising a polymer shell 2 are not aggregating over time (compare to Fig. 4). The nanoparticles 1 are suspended in an aqueous solution 12 and have been prepared according to the method according to the invention. The such obtained nanoparticles 1 comprise a PLLA coating that encloses a structured plurality 40 of triphenylalanines 41, that give rise to the detectable SHG signal. Image A) has been taken at time 0 minutes, image B) after 20 minutes, image C) after 40 minutes and image D) after 60 minutes. As can be seen, the SHG signal is not clustering over the course of time, which would indicate aggregation. Thus, the polymer shell 2 indeed prevents the aggregation of the self-assembled oligopeptides 4.

Figure 8 shows the molecular structures of cyclo(-D-Trp-Tyr) 50 on the top panel and cyclo(-Phe-Phe) 51 on the bottom panel.

### Example:

In the following a brief, non-limiting example of the preparation of a suspension of nanoparticles according to the invention is given:
- Dissolve 5 mg Phe-Phe-Phe and 30 mg Poly-Lactic Acid in 3 ml Chloroform.
- Mix the peptide-polymer solution with 10 ml SDS aqueous solution (1 mg/ml) as surfactant.
- After stirring the mixture for 1 hour, sonicate the sample using a Probe Sonicator for 2 minutes at 70% power.
- stir the suspension overnight at 37 °C to evaporate chloroform and so that the aqueous suspension of nanoparticles according to the invention is obtained.

While the actual steps remain particularly the same, the concentrations of oligopeptide, polymer and surfactant might be different when using a different oligopeptide.

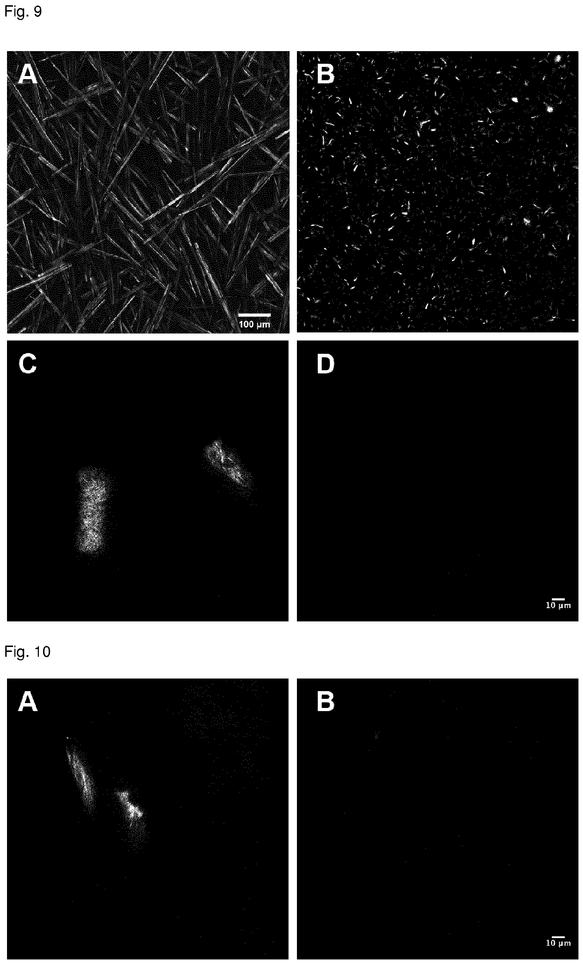

Fig. 9 shows various SHG images. The white regions in the images indicate the presence of an SHG signal, wherein the black regions indicate the absence of an SHG signal. Panel A: State of the art large scale peptide nanotubes using diphenylalanine (FF) peptide. Such large scale assemblies are formed when no shell layer is provided to inhibit the growth of the oligopeptides. Panel B: In contrast to the state of the art, encapsulated triphenylalanine (FFF) oligopeptide based on the emulsion-solvent evaporation method according to the invention form stable nanoparticles that generate a SHG signal upon illumination. Panel C: LIVAGK peptide assemblies described by [4] show large-scale peptide assemblies. Panel D: Nonetheless, when LIVAGK peptides are encapsulated, the SHG signal vanishes.

Fig. 10 shows two SHG images of LIVAGK assemblies in two different concentrations. Panel A: LIVAGK peptides' SHG signal at a concentration of 100 mg/ml. Panel B: LIVAGK peptides' SHG signal at a concentration of 10 mg/ml. In panel B no SHG signal can be observed, as the aggregated peptides have disassembled.

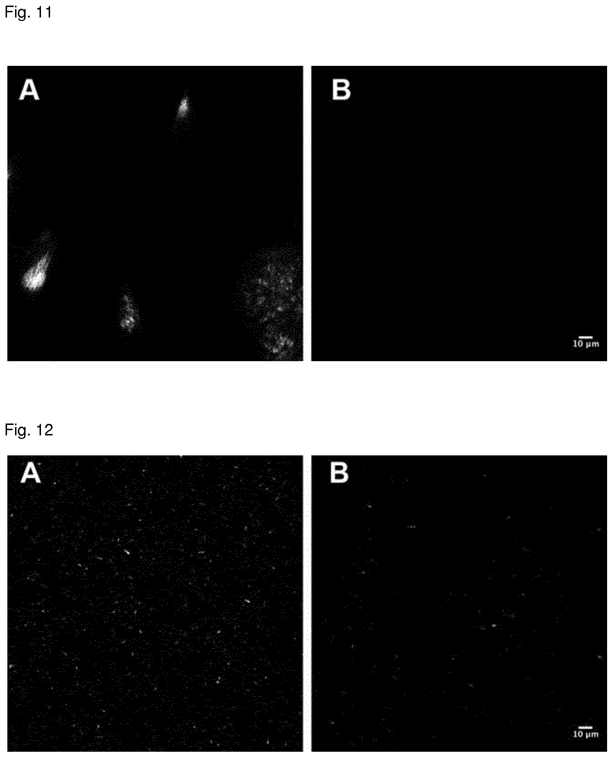

Fig. 11 shows two SHG images of LIVAGK assemblies in a solution with a pH-value of 7 (panel A) or pH 4 (panel B) at a concentration of 100 mg/ml. While in panel A the peptides are still assembled and give rise to an SHG signal, the peptides disassemble at pH 4, such that the SHG signal cannot be observed anymore. The disassembly occurs within minutes (~5 minutes) after incubation in pH 4.

Fig. 12 shows two SHG images of nanoparticles according to the invention. Panel A: shows the nanoparticles' SHG signal at a concentration of 1.5 mg/ml. Panel B shows the SHG signal of the nanoparticles at a concentration of 0.15 mg/ml. The nanoparticles do not suffer from dissociation or disassembly but remain stable. The SHG signal persists.

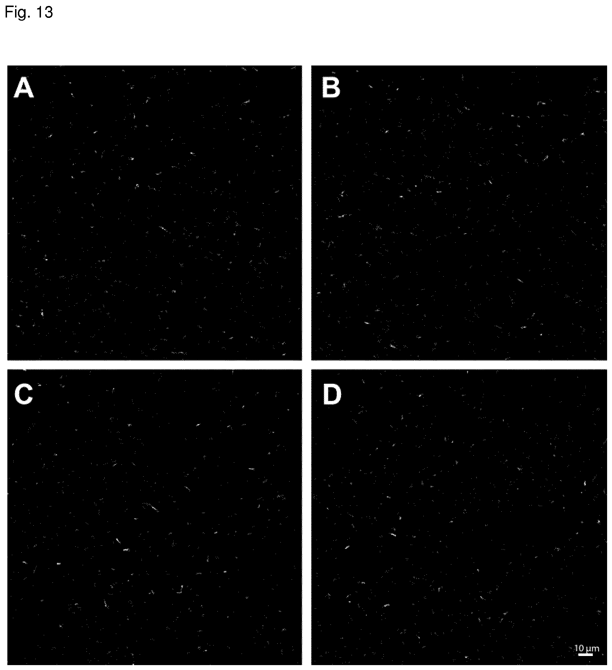

Fig. 13 shows SHG images of the nanoparticles according to the invention in various solutions with different pH-values. Panel A: SHG signal after incubation of the nanoparticles for 72 hours in a buffer at pH 7. Same as in panel A, but at pH 6. Same as in panel A, but at pH 5. Panel D: same as in panel A, but at pH 4.

### References:

[1] Boyd, R. W. Nonlinear optics (Academic Press, 2013)
[2] Kholkin, A., et al. "Strong piezoelectricity in bioinspired peptide nanotubes", ACS Nano 4, 610-614 (2010)
[3] Handelmann, A. et al. "Nonlinear optical bioinspired peptides nanostructures" Advanced Optical Materials 1, 875-884 (2013)
[4] WO 2016/007091 A1

## Claims

1. Biodegradable, biocompatible, water-suspensable nanoparticle (1), for generating a second-harmonic light signal upon illumination, comprising
- a shell layer (2) comprising a biodegradable polymer (3), wherein the shell layer (2) encloses
- a plurality (40) of oligopeptides (4), wherein the plurality (40) of oligopeptides (4) is structured and lacks an inversion symmetry such that a second-harmonic light signal is generated by the structured plurality of oligopeptides upon illumination of the nanoparticle (1) with light.

2. Nanoparticle according to claim 1, wherein the structured plurality (40) of oligopeptides (4) comprises or consists of self-assembling oligopeptides (4), wherein the structured plurality (40) of oligopeptides (4) is arranged in a self-assembled structure.

3. Nanoparticle according to claim 1 or 2, wherein the plurality (40) of oligopeptides (4) comprises or consists of at least one of:
- cyclo(-D-Trp-Tyr) (50),
- Trp-Phe,
- Phe-Phe-Phe (41),
- Phe-Phe,
- Ala-Ala-Ala-Ala-Ala,
- cyclo(-Phe-Phe) (51),
- Leu-Phe,
- Leu-Leu.

4. Nanoparticle according to one of the preceding claims, wherein the plurality (40) of oligopeptides (4) comprises at least one structured and shape-persistent region exhibiting a high degree of internal order.

5. Nanoparticle according to one of the preceding claims, wherein the plurality (40) of oligopeptides (4) is structured by means of non-covalent interactions, particularly by hydrophobic interactions and/or hydrogen bonds.

6. Nanoparticle according to one of the preceding claims, wherein the biodegradable polymer (3) is one of:
- a poly(L-lactide) (PLLA),
- a polyglycolide and a polylactic polyglycolic copolymer,
- a hydroxy-terminated poly(ε-caprolactone)-polyether,
- a polycaprolactone,
- a poly[(D,L-lactide)-co-glycolide] (PLGA),
- a polyacrylamide,
- a poly(orthoester),
- a biodegradable polyurethane,
- a polycyanoacrylate polymer,
- a poly(γ-glutamic acid) (γ-PGA),
- a phenylalanine ethyl ester.

7. Nanoparticle according to any of the preceding claims, wherein the plurality of oligopeptides is crystalized in crystal unit cells, wherein the second-harmonic light signal is generated from the crystal unit cells upon illumination of the nanoparticle.

8. Nanoparticle according to one of the preceding claims, wherein the polymer shell (2) comprises functional chemical groups or peptides for targeting biological binding sites or for binding to specific epitopes, particularly to cancer cell receptors.

9. Method for preparing an aqueous suspension of biodegradable, water-suspendable nanoparticles (1) according to claim 1, wherein the nanoparticles (1) generate a second-harmonic signal upon illumination, comprising the steps:
- providing an organic phase (10) comprising an organic, particularly water-immiscible solvent with oligopeptides (4) and a biodegradable polymer (3) and/or monomers that form the polymer (3) upon polymerization, wherein the oligopeptides (4) assemble in at least one structured plurality (40) of oligopeptides (4) in each nanoparticle (1) when the nanoparticle (1) is prepared, wherein said structured plurality (40) of oligopeptides (4) generates a second-harmonic signal upon illumination,
- providing a continuous aqueous phase (12) comprising an aqueous solution with a surfactant (11),
- preparing (100) a miniemulsion (14) of the organic phase (10) and the aqueous phase (12), wherein the miniemulsion (14) comprises micelles (13) of the organic phase (10) emulsified in the aqueous phase (12),
- removing (101) the organic solvent from the miniemulsion (14) wherein the removal of the organic solvent leads to the formation of nanoparticles (1) according to one of the claims 1 to 8 in the aqueous solution (12).

10. Method according to claim 9, wherein the miniemulsion (14) is prepared by applying shear-forces to the mixed solution of the organic phase (10) and aqueous phase (12), wherein the shear-forces are applied by sonication of the mixed solution.

11. Method according to claim 9 or 10, wherein the organic solvent is removed (101) from the miniemulsion (14) by evaporating the organic solvent.

12. Method according to one of the claims 9 to 11, wherein the organic solvent is chloroform, the oligopeptide (4) is or comprises triphenylalanine (41) and the polymers (3) comprise or consist of Poly-Lactic Acid and the aqueous phase (12) comprises sodium dodecyl sulfate as surfactant (11).

13. Method according to one of the claims 9 to 12, wherein the monomers are polymerized in a polymerization step.

14. Method for second-harmonic generation imaging of a sample obtained from a person or an animal comprising a nanoparticle (1) according to one of the claims 1 to 8, comprising the steps of:
- providing a sample with the nanoparticle (1),
- illuminating the sample with light comprising a first wavelength, particularly 1064 nm,
- detecting light at half the wavelength of the first wavelength.

15. Use of a nanoparticle according to claim 1 to 8 for second-harmonic generation imaging of biological samples, cells, tissue preparations or tissue by applying the imaging method according to claim 14.

## Patentansprüche

1. Biologisch abbaubares, biokompatibles, wasser-suspendierbares Nanopartikel (1) zum Erzeugen eines frequenzverdoppelten Lichtsignals bei Belichtung, umfassend
- eine Hüllschicht (2), umfassend ein biologisch abbaubares Polymer (3), wobei die Hüllschicht (2) Folgendes umschließt
- eine Vielzahl (40) von Oligopeptiden (4), wobei die Vielzahl (40) von Oligopeptiden (4) strukturiert ist und keine Inversionssymmetrie aufweist, so dass ein frequenzverdoppeltes Lichtsignal durch die strukturierte Vielzahl von Oligopeptiden bei Belichtung des Nanopartikels (1) mit Licht erzeugt wird.

2. Nanopartikel gemäß Anspruch 1, wobei die strukturierte Vielzahl (40) von Oligopeptiden (4) selbstassemblierende Oligopeptide (4) umfasst oder aus diesen besteht, wobei die strukturierte Vielzahl (40) von Oligopeptiden (4) in einer selbstassemblierenden Struktur angeordnet ist.

3. Nanopartikel gemäß Anspruch 1 oder 2, wobei die Vielzahl (40) von Oligopeptiden (4) mindestens eines der folgenden umfasst oder daraus besteht:
- cyclo(-D-Trp-Tyr) (50),
- Trp-Phe,
- Phe-Phe-Phe (41),
- Phe-Phe,
- Ala-Ala-Ala-Ala-Ala,
- cyclo(-Phe-Phe) (51),
- Leu-Phe,
- Leu-Leu.

4. Nanopartikel gemäß einem der vorhergehenden Ansprüche, wobei die Vielzahl (40) von Oligopeptiden (4) mindestens einen strukturierten und formstabilen Bereich umfasst, der einen hohen Grad an innerer Ordnung aufweist.

5. Nanopartikel gemäß einem der vorhergehenden Ansprüche, wobei die Vielzahl (40) der Oligopeptide (4) mittels nicht-kovalenter Wechselwirkungen, insbesondere durch hydrophobe Wechselwirkungen und/oder Wasserstoffbrückenbindungen, strukturiert ist.

6. Nanopartikel gemäß einem der vorhergehenden Ansprüche, wobei das biologisch abbaubare Polymer (3) eines ist aus:
- ein Poly(L-Lactid) (PLLA),
- ein Polyglykolid und ein Polymilchpolyglykol-Copolymer,
- ein Hydroxy-terminierter Poly(ε-caprolacton)-Polyether,
- ein Polycaprolacton,
- ein Poly[(D, L-lactid)-Co-Glycolid] (PLGA),
- ein Polyacrylamid,
- ein Poly(orthoester),
- ein biologisch abbaubares Polyurethan,
- ein Polycyanoacrylat-Polymer,
- ein Poly(y-glutaminsäure) (y-PGA),
- ein Phenylalanin-Ethylester.

7. Nanopartikel gemäß einem der vorhergehenden Ansprüche, wobei die Vielzahl von Oligopeptiden in Kristallelementarzellen kristallisiert ist, wobei das frequenzverdoppelte Lichtsignal von den Kristallelementarzellen bei Belichtung des Nanopartikels erzeugt wird.

8. Nanopartikel gemäß einem der vorhergehenden Ansprüche, wobei die Polymerhülle (2) funktionelle chemische Gruppen oder Peptide zum Ausrichten auf biologische Bindungsstellen oder zum Binden an spezifische Epitope, insbesondere an Krebszellrezeptoren, umfasst.

9. Verfahren zum Herstellen einer wässrigen Suspension von biologisch abbaubaren, wasser-suspendierbaren Nanopartikeln (1) gemäß Anspruch 1, wobei die Nanopartikel (1) bei Belichtung ein frequenzverdoppeltes Signal erzeugen, umfassend die Schritte:
- Bereitstellen einer organischen Phase (10), umfassend ein organisches, insbesondere nicht wasservermischbares Lösungsmittel mit Oligopeptiden (4) und einem biologisch abbaubaren Polymer (3) und/oder Monomeren, die das Polymer (3) bei Polymerisation bilden, wobei sich die Oligopeptide (4) bei der Präparation des Nanopartikels (1) in jedem Nanopartikel (1) zu mindestens einer strukturierten Vielzahl (40) von Oligopeptiden (4) zusammensetzen, wobei die besagte strukturierte Vielzahl (40) von Oligopeptiden (4) bei Belichtung ein frequenzverdoppeltes Signal erzeugt,
- Bereitstellen einer kontinuierlichen wässrigen Phase (12), umfassend eine wässrige Lösung mit einem Tensid (11),
- Präparieren (100) einer Miniemulsion (14) der organischen Phase (10) und der wässrigen Phase (12), wobei die Miniemulsion (14) Mizellen (13) der organischen Phase (10) umfasst, die in der wässrigen Phase (12) emulgiert sind,
- Entfernen (101) des organischen Lösungsmittels aus der Miniemulsion (14), wobei das Entfernen des organischen Lösungsmittels zur Bildung von Nanopartikeln (1) nach einem der Ansprüche 1 bis 8 in der wässrigen Lösung (12) führt.

10. Verfahren gemäß Anspruch 9, wobei die Miniemulsion (14) durch Anlegen von Scherkräften an die gemischte Lösung aus der organischen Phase (10) und der wässrigen Phase (12) präpariert wird, wobei die Scherkräfte durch Beschallen der gemischten Lösung angelegt werden.

11. Verfahren gemäß Anspruch 9 oder 10, wobei das organische Lösungsmittel aus der Miniemulsion (14) durch Verdampfen des organischen Lösungsmittels entfernt wird (101).

12. Verfahren gemäß einem der Ansprüche 9 bis 11, wobei das organische Lösungsmittel Chloroform ist, das Oligopeptid (4) Triphenylalanin (41) ist oder umfasst und die Polymere (3) aus Polymilchsäure bestehen oder diese umfassen und die wässrige Phase (12) Natriumdodecylsulfat als Tensid (11) umfasst.

13. Verfahren nach einem der Ansprüche 9 bis 12, wobei die Monomere in einem Polymerisationsschritt polymerisiert werden.

14. Verfahren zur Frequenzverdoppelten-Bildgebung einer von einer Person oder einem Tier erhaltenen Probe, umfassend ein Nanopartikel (1) nach einem der Ansprüche 1 bis 8, umfassend die Schritte:
- Bereitstellen einer Probe mit dem Nanopartikel (1),
- Belichten der Probe mit Licht einer ersten Wellenlänge, insbesondere 1064 nm,
- Licht mit der halben Wellenlänge der ersten Wellenlänge detektieren.

15. Verwendung eines Nanopartikels gemäß einem der Ansprüche 1 bis 8 zur Frequenzverdoppelten-Bildgebung von biologischen Proben, Zellen, Gewebepräparaten oder Gewebe durch Anwenden des Bildgebungsverfahrens gemäß Anspruch 14.

## Revendications

1. Nanoparticule biodégradable, biocompatible, suspensible dans l'eau (1) pour générer un signal lumineux à deuxième harmonique dès l'éclairement, comprenant
- une couche de coque (2) comprenant un polymère biodégradable (3), dans laquelle la couche de coque (2) renferme
- une pluralité (40) d'oligopeptides (4), dans laquelle la pluralité (40) d'oligopeptides (4) est structurée et dépourvue d'une symétrie d'inversion de sorte qu'un signal lumineux à deuxième harmonique est généré par la pluralité structurée d'oligopeptides dès l'éclairement de la nanoparticule (1) avec de la lumière.

2. Nanoparticule selon la revendication 1, dans laquelle la pluralité structurée (40) d'oligopeptides (4) comprend ou est constituée d'oligopeptides à autoassemblage (4), dans laquelle la pluralité structurée (40) d'oligopeptides (4) est agencée dans une structure autoassemblée.

3. Nanoparticule selon la revendication 1 ou 2, dans laquelle la pluralité (40) d'oligopeptides (4) comprend ou est constituée d'au moins un parmi :
- cyclo(-D-Trp-Tyr) (50),
- Trp-Phe,
- Phe-Phe-Phe (41),
- Phe-Phe,
- Ala-Ala-Ala-Ala-Ala,
- cyclo(-Phe-Phe) (51),
- Leu-Phe,
- Leu-Leu.

4. Nanoparticule selon une des revendications précédentes, dans laquelle la pluralité (40) d'oligopeptides (4) comprend au moins une région structurée et à forme persistante présentant un degré élevé d'ordre interne.

5. Nanoparticule selon une des revendications précédentes, dans laquelle la pluralité (40) d'oligopeptides (4) est structurée au moyen d'interactions non covalentes, notamment par des interactions hydrophobes et/ou liaisons hydrogène.

6. Nanoparticule selon une des revendications précédentes, dans laquelle le polymère biodégradable (3) est un parmi :
- un acide poly(L-lactique) (PLLA),
- un acide polyglycolique et un copolymère polyglycolique polylactique,
- un poty(ε-caprotactone)-polyéther à terminaison hydroxy,
- une polycaprolactone,
- un acide poly[(D,L-lactique)-co-glycolique] (PLGA),
- un polyacrylamide,
- un poly(orthoester),
- un polyuréthane biodégradable,
- un polymère de polycyanoacrylate,
- un acide poly(γ-glutamique) (y-PGA),
- un ester éthylique de phénylalanine.

7. Nanoparticule selon l'une quelconque des revendications précédentes, dans laquelle la pluralité d'oligopeptides est cristallisée en motifs élémentaires cristallins, dans laquelle le signal lumineux à deuxième harmonique est généré à partir des motifs élémentaires cristallins dès l'éclairement de la nanoparticule.

8. Nanoparticule selon une des revendications précédentes, dans laquelle la coque polymère (2) comprend des groupes chimiques fonctionnels ou peptides pour cibler des sites de liaison biologiques ou pour se lier à des déterminants antigéniques spécifiques, notamment à des récepteurs de cellule cancéreuse.

9. Procédé de préparation d'une suspension aqueuse de nanoparticules biodégradables, suspensibles dans l'eau (1) selon la revendication 1, dans lequel les nanoparticules (1) génèrent un signal à deuxième harmonique dès l'éclairement, comprenant les étapes :
- fournir une phase organique (10) comprenant un solvant organique, notamment immiscible à l'eau avec des oligopeptides (4) et un polymère biodégradable (3) et/ou des monomères qui forment le polymère (3) dès la polymérisation, dans lequel les oligopeptides (4) s'assemblent en au moins une pluralité structurée (40) d'oligopeptides (4) dans chaque nanoparticule (1) lorsque la nanoparticule (1) est préparée, dans lequel ladite pluralité structurée (40) d'oligopeptides (4) génère un signal à deuxième harmonique dès l'éclairement,
- fournir une phase aqueuse continue (12) comprenant une solution aqueuse avec un tensioactif (11),
- préparer (100) une mini-émulsion (14) de la phase organique (10) et la phase aqueuse (12), dans lequel la mini-émulsion (14) comprend des micelles (13) de la phase organique (10) émulsifiées dans la phase aqueuse (12),
- éliminer (101) le solvant organique de la mini-émulsion (14), dans lequel l'élimination du solvant organique conduit à la formation de nanoparticules (1) selon une des revendications 1 à 8 dans la solution aqueuse (12).

10. Procédé selon la revendication 9, dans lequel la mini-émulsion (14) est préparée en appliquant des forces de cisaillement à la solution mélangée de la phase organique (10) et la phase aqueuse (12), dans lequel les forces de cisaillement sont appliquées par sonification de la solution mélangée.

11. Procédé selon la revendication 9 ou 10, dans lequel le solvant organique est éliminé (101) de la mini-émulsion (14) par évaporation du solvant organique.

12. Procédé selon une des revendications 9 à 11, dans lequel le solvant organique est le chloroforme, l'oligopeptide (4) est ou comprend de la triphénylalanine (41) et les polymères (3) comprennent ou sont constitués d'acide poly-lactique, et la phase aqueuse (12) comprend du dodécylsulfate de sodium en tant que tensioactif (11).

13. Procédé selon une des revendications 9 à 12, dans lequel les monomères sont polymérisés dans une étape de polymérisation.

14. Procédé d'imagerie par génération de deuxième harmonique d'un échantillon obtenu à partir d'une personne ou d'un animal comprenant une nanoparticule (1) selon une des revendications 1 à 8, comprenant les étapes consistant à :
- fournir un échantillon avec la nanoparticule (1),
- éclairer l'échantillon avec de la lumière comprenant une première longueur d'onde, notamment 1064 nm,
- détecter de la lumière à la moitié de la longueur d'onde de la première longueur d'onde.

15. Utilisation d'une nanoparticule selon les revendications 1 à 8 pour l'imagerie par génération de deuxième harmonique d'échantillons biologiques, de cellules, de préparations de tissu ou de tissu en appliquant le procédé d'imagerie selon la revendication 14.


### SEQUENCE LISTING

<110> ETH Zürich Max-Planck-Gesellschaft zur Förderung der Wissenschaften e.V.
<120> Biodegradable, Second-Harmonic-Generating Nanoprobe for Biomedical Imaging Applications
<130> eth156wo
<160> 1
<170> PatentIn version 3.5
<210> 1
   <211> 5
   <212> PRT
   <213> Artificial Sequence
<220>
   <223> Monomer for SHG nanorod
<400> 1
   Ala Ala Ala Ala Ala
   1               5